## Europäisches Patentamt
### European Patent Office
### Office européen des brevets

(11) Veröffentlichungsnummer: **0 415 183 A2**

# EUROPÄISCHE PATENTANMELDUNG

(21) Anmeldenummer: 90115649.7

(22) Anmeldetag: 16.08.90

(51) Int. Cl.5: **A61L 15/22**, A61L 25/00, C08J 3/24, C08K 5/54

(30) Priorität: 31.08.89 DE 3928858

(43) Veröffentlichungstag der Anmeldung:
06.03.91 Patentblatt 91/10

(84) Benannte Vertragsstaaten:
AT BE DE ES FR GB IT NL SE

(71) Anmelder: Beiersdorf Aktiengesellschaft
Unnastrasse 48
W-2000 Hamburg 20(DE)

(72) Erfinder: Pietsch, Hanns, Dr.
Mittelweg 25
W-2000 Hamburg 13(DE)
Erfinder: Borgschulte, Katrin, Dr.
Eppendorfer Weg 168
W-2000 Hamburg 20(DE)
Erfinder: Steinert, Hans-Jürgen, Dr.
Fährhausstrasse 14
W-2000 Hamburg 76(DE)
Erfinder: Wesselkamp, Ingrid
Krögerstrasse 54
W-2000 Hamburg 73(DE)
Erfinder: Trotter, Sebastian
Am Heidberg 13
W-2110 Buchholz(DE)

(54) Vernetzte Hydrogele und Verfahren zu deren Herstellung.

(57) Vernetzte Hydrogele, insbesondere für medizinische Anwendungen, dadurch erhältlich, daß natürliche oder synthetische makromolekulare Verbindung mit hydrophilen Gruppen durch Alkoxysilylverbindungen bei pH-Werten im neutralen bis sauren Bereich in wäßrigem Medium vernetzt werden, wobei unter hydrophilen Gruppen Hydroxylgruppen ( -OH),
Carboxylgruppen ( -COOH),
Amidgruppen ( -CONRR′, mit R,R′ gewählt aus H, Methyl, Ethyl),
Aminogruppen (primäre, sekundäre, tertiäre sowie quaternäre) und
Sulfonsäuregruppen
zu verstehen sind.

EP 0 415 183 A2

## VENETZTE HYDROGELE UND VERFAHREN ZU DEREN HERSTELLUNG

Die vorliegende Erfindung betrifft vernetzte Hydrogele, insbesondere vernetzte Hydrogele für medizinische Anwendungen, sowie Verfahren für ihre Herstellung.

Hydrogele sind makromolekulare, natürliche oder synthetische Stoffe, die aufgrund eines hohen Gehaltes an hydrophilen Gruppen in der Lage sind, Wasser absorptiv zu binden. Die Wasseraufnahmekapazität vieler Hydrogele beträgt das Mehrfache des Eigengewichtes der wasserfreien Substanz.

Hydrogele werden in vielfältiger Form in der Medizin eingesetzt. Besonders geeignet sind sie in der Wundversorgung; sie können

* Wunden vor der Austrocknung schützen
* Wundsekret aufsaugen
* als Matrix für Wirkstoffe aller Art dienen
* als Basis für die Besiedelung mit autologen oder heterologen Hautzellen dienen

Hydrogele werden in Form von Pulvern, Granulaten, Folien, Folienstücken, Schäumen oder Schaumstücken verwendet.

Die Hydrogele des Standes der Technik haben jedoch verschiedene Nachteile:

Aufgrund ihrer Hydrophilie sind die meisten in Frage kommenden Stoffe wasserlöslich. Dies ist meist unerwünscht, weil derartige Produkte nicht formstabil sind. Außerdem lösen sich derartige Produkte in unerwünschter Weise am Einsatzort auf und stehen dann für den vorgesehenen Zweck nicht mehr zur Verfügung.

Andere Produkte zeichnen sich durch starke Polymervernetzung aus. Dadurch werden zwar manche der Nachteile der vorgenannten Stoffklasse vermieden. Die Quellbarkeit dieser Stoffe ist jedoch weitgehend eingeschränkt oder verloren. Dadurch sind auch Stoffe dieser Art allenfalls bedingt einsetzbar für medizinische Anwendungen.

Die vorgeschilderten Nachteile des Standes der Technik lassen sich wie folgt zusammenfassen:

Je nach Vernetzungsgrad resultiert eine mehr oder weniger starke Quellfähigkeit. Je stärker die Vernetzung desto geringer die Quellfähigkeit und je geringer die Vernetzung, desto größer die Quellfähigkeit. So wird das Quellungsvermögen von vernetzten Makromolekülen auch zur Charakterisierung des Vernetzungsgrades benutzt.

$$Q = \frac{(G_2 - G_1) * 100}{G_1}$$

mit

$G_1$ = Gewicht des Hydrogels vor der Quellung

$G_2$ = Gewicht des Hydrogels nach der Quellung

$Q$ = "Quellwert" (in %).

Typische Quellwerte belaufen sich zwischen 30 und 700 %

In EP-A-0 097 846 werden Wundbehandlungsmittel auf der Basis von Hydrogelen beschrieben. Dabei wird Gelatine in fester Form als Pulver, Schuppen oder Blatt in einer Zwei-Phasen Reaktion mit Vernetzern wie Formaldehyd, Glyoxal, Glutarsäuredialdehyd, Dicarbonsäurechloriden und/oder Diisocyanaten umgesetzt.

Der Vernetzer wirkt dabei auf die gequollene, nicht gelöste Gelatine ein.

Dieses Verfahren und die daraus erhaltenen Produkte sind mit erheblichen Nachteilen behaftet, da die verwendeten Vernetzer erhebliche Zellschädigungen bewirken können.

Zudem ist das Verfahren nicht oder nur schwer reproduzierbar. Die Vernetzung hängt nicht nur von der Konzentration der verwendeten Vernetzer ab, sondern auch von Parametern wie Temperatur und Einwirkzeit der Reaktanden. Ferner sind die effektive Oberfläche und das mittlere Molekulargewicht der handelsüblichen Gelatinearten deutlichen Schwankungen unterworfen, so daß auch die Eigenschaften des vernetzten Hydrogels schwer voraussehbar sind.

Weiterhin ist in EP-A-0 097 846 ein Verbandmaterial aus Hydrogelen auf Polyvinylakoholbasis beschrieben. Als Vernetzer wird Formaldehyd eingesetzt, der, wie eingangs erwähnt, physiologisch bedenklich ist.

In US-4,093,673 werden organische Alkoxysilan-Vernetzer beschrieben, die in der Lage sind, in Gegenwart von geeigneten Katalysatoren hydroxylgruppenhaltige Polymere zu vernetzen. Solche Katalysatoren sind: Organische Säuren, Metallsalze von organischen Säuren oder organische Basen z.B.: p-Toluolsulfonsäure, n-Butylphosphorsäure, Zinn naphtenat, Zinn benzoat, Zinn octoat, Zinn butyrat, Zinn-2-ethylhexanoat, Dibutylzinn dioctoat, Dibutylzinn dilaurat, Dibutylzinn diacetat, Eisenstearat, Bleioctoat, Isophorondiamin, Methylendianilin, Imidazol.

Alle genannten Vernetzungskatalysatoren sind toxisch oder zelltoxisch und nicht für die Anwendung im menschlichen Körper auf offenen Wunden oder Schleimhäuten geeignet.

Es war daher die Aufgabe der Erfindung, vernetzte Hydrogele zu entwickeln, welche nicht die Nachteile des Standes der Technik haben und für den Kontakt mit offenen Wunden, Blut oder für den dauernden Verbleib als Implantat im menschlichen Körper geeignet sind. Außerdem sollten die Kydrogele nach reproduzierbaren Verfahren wirtschaftlich herstellbar sein.

Überraschend wurde gefunden, daß mehrwertige Alkoxysilylverbindungen in der Lage sind, bei pH-Werten zwischen 4 - 7, im wäßrigen Medium ohne zusätzlichen Vernetzungskatalysator, makromolekulare Verbindungen mit hydrophilen Gruppen zu vernetzen, wobei Hydrogele mit den erstrebten Eigenschaften entstehen.

Im folgenden sind unter hydrophilen Gruppen zu verstehen:

Hydroxylgruppen ( -OH)

Carboxylgruppen ( -COOH)

Amidgruppen ( -CONRR$'$, mit R,R$'$ gewählt aus H, Methyl, Ethyl).

Aminogruppen (primäre, sekundäre, tertiäre sowie quaternäre)

Sulfonsäuregruppen .

Als makromolekulare Verbindungen mit hydrophilen Gruppen werden bevorzugt Gelatine, lösliches Kollagen, Polyvinylalkohol, Poly-2-hydroxyethylmethacrylat, partiell verseiftes Polyvinylacetat, Methylcellulose, Hydroxypropylmethylcellulose, Hydroxyethylcellulose, Dextran, Carrageenan, Alginate, Maleinsäureanhydrid-Vinylether-Copolymerisate, Chitosan, Carboxymethyl-Chitin, Polyacrylsäure und/oder Polymethacrylsäure als Homo- oder Copolymerisate jeweils allein oder in Mischung verwendet.

Besonders bevorzugt sind Gelatine, Polyvinylalkohol und Chitosan.

Ganz besonders bevorzugt sind Polyvinylalkohole mit durchschnittlichen Molekulargewichten von 15.000 - 150.000 Dalton.

Von den makromolekularen Stoffen können auch Copolymere verwendet werden, sofern sie mindestens zu 50 Mol-% Monomer- Einheiten mit hydrophilen Gruppen enthalten. Bevorzugt werden die hydroxylgruppenhaltigen makromolekularen Verbindungen; doch sind auch carboxylgruppenhaltige oder amidgruppenhaltige Verbindungen vorteilhaft. Diese können günstig in Kombination mit hydroxylgruppenhaltigen Verbindungen, als Polymerengemisch oder Copolymerisat eingesetzt werden.

Solche carboxylgruppenhaltigen Verbindungen sind bevorzugt Polyacrylsäure, Polymethacrylsäure oder deren Copolymerisate und Maleinsäure-Methylvinylether-Copolymerisat. Bei letzterem entstehen aus Anhydridstrukturen durch Reaktion mit Wasser Carboxylgruppen.

Die carboxylgruppenhaltigen makromolekularen Verbindungen können wegen ihrer Säurefunktion auch zur pH-Wert-Regulierung eingesetzt werden. Daß dies gegebenenfalls von Vorteil ist, wird weiter unten ausgeführt.

Die erfindungsgemäßen Vernetzer sind bevorzugt Alkoxysilylverbindungen, besonders bevorzugt gewählt aus der Gruppe der Verbindungen der folgenden allgemeinen Formel:

$HRN-(CH_2-CH_2-NR')_n-(CH_2)_m-Si(OR'')_3$

mit R, R$'$ gewählt aus der Gruppe H, Methyl oder Ethyl

und R$''$ gewählt aus der Gruppe Methyl, Ethyl

wobei n Werte von 0 - 5

und m Werte von 1 - 4 annehmen kann,

wie beispielsweise

3-Aminopropyltriethoxysilan, N-Aminoethylaminopropyltrimethoxysilan, "Triaminosilan" ($H_2N-CH_2-CH_2-NH-CH_2-CH_2-NH-CH_2-CH_2-CH_2-Si(OCH_3)_3$), N-Methyl-3-aminopropyltrimethoxysilan.

Günstig sind aber auch

3-Ureidopropyltriethoxysilan,

3- (4, 5-Dihydroimidazol-1-yl)-propyltriethoxysilan,

3-Methacryloxypropyltriethoxysilan,

3-Glycidyloxypropyltrimethoxysilan, Vinyltriethoxysilan,

Vinyltrimethoxysilan,

EP 0 415 183 A2

Tetramethoxysilan oder
Tetraethoxysilan.

Von den genannten Verbindungen hat sich das 3-Aminopropyltriethoxysilan als Vernetzer mit besonders guter physiologischer Verträglichkeit herausgestellt.

Die damit erhaltenen vernetzten Hydrogele zeichnen sich durch gute mechanische Festigkeit und chemische Stabilität aus.

Bevorzugt wird die Vernetzung der hydrophilen makromolekularen Verbindung ohne Katalysator durchgeführt. Es hat sich jedoch gezeigt, daß anorganische Säuren wie Phosphorsäure, Salzsäure oder Schwefelsäure oder organische Säuren wie Essigsäure, Milchsäure, Zitronensäure, Ascorbinsäure oder Salicylsäure die Vernetzung in einem pH-Bereich zwischen 4 - 7 katalysieren.

Dabei nehmen die Salzsäure und die Essigsäure eine Sonderstellung ein, da sie flüchtig sind. Sorgt man dafür, daß durch geeignete Lagerung diese Säuren verdampfen können, ist es möglich, ein vernetztes Hydrogel ohne Säurerückstände zu erhalten.

Eine weitere Sonderstellung nehmen die Ascorbinsäure und Salicylsäure ein, weil sie die Wundheilung fördern. Von den nichtflüchtigen und nicht die Heilung fördernden Säuren wiederum ist die Phosphorsäure bevorzugt.

Die vernetzten Hydrogele können bis zu 60%, bezogen auf die Trockensubstanz, an Weichmachern, vorzugsweise flüssigen Polyolen enthalten, wobei das bevorzugte Polyol mit der geringsten Zelltoxizität das Glycerin ist. Andere Polyole, die an sich eine gewisse Zelltoxizität aufweisen, können dennoch vorteilhaft eingesetzt werden, wenn sie besser verträglich mit den vernetzten Hydrogelen sind, zum Beispiel: Ethylenglycol, flüssige Polyethylenglycole verschiedener Molmasse, Propylenglycol-1,2, flüssige Polypropylenglycole-1,2 verschiedener Molmasse, Propylenglycol-1,3, flüssige Polypropylenglycole-1,3 verschiedener Molmasse, Zitronensäuretriethyl- oder trimethylester, Milchsäuremethyl- oder -ethylester, Glycolsäuremethyl- oder -ethylester oder 2-Ethyl-2(hydroxymethyl)-1,3-propandiol. Diese Verbindungen schützen die erfindungsgemäßen Hydrogele vor dem Austrocknen. Dies ist insbesondere dann günstig, wenn das fertige Hydrogel nicht wasserdicht oder wasserdampfundurchlässig verpackt wird.

Die bevorzugten erfindungsgemäßen Zubereitungen enthalten, bezogen auf die wasserfreie Substanz,
40 - 95 Gew.-% an makromolekularen Verbindungen mit hydrophilen Gruppen
4 - 60 Gew.-% an Weichmachern
0,1 - 5,0 Gew.-% an Alkoxysilylgruppenhaltigen Vernetzern
0 - 5,0 Gew.-% an Säuren,

Die Herstellung der erfindungsgemäßen vernetzten Hydrogele erfolgt derart, daß zunächst die makromolekulare Verbindung mit der erforderlichen Menge Wasser unter Rühren bei 40 - 90 °C gelöst wird und sodann die anderen Verbindungen, zuletzt der Vernetzer, dazugegeben werden. Diese Lösung kann vorteilhaft warm verarbeitet werden.

Es ist von besonderem Vorteil, daß die Vernetzungsreaktion langsam verläuft, so daß es während des Herstellungsverfahrens nicht zu Vergelungen kommt. Die Menge des Vernetzers hängt von dem gewünschten Vernetzungsgrad ab und liegt zwischen 0,1 - 5,0 Gewichtsprozent, bezogen auf die Gesamtmenge der wasserfreien Substanzen. Je nach Reaktivität der verwendeten hydroxylgruppenhaltigen makromolekularen Verbindung und der Temperatur kann die Vernetzungsreaktion bis zu 6 Monaten dauern. Man erkennt die abgeschlossene Reaktion daran, daß das Hydrogel nicht mehr in Wasser löslich ist und der angestrebte Quellwert erreicht ist. Das auf diese Weise gewonnene rohe Hydrogel kann entweder bei Raumtemperatur oder bei höheren Temperaturen, vorteilhaft bei Temperaturen von 60 - 95 °C getrocknet werden.

Es ist möglich und zweckmäßig, die Lösung des rohen Hydrogels zu dünnen Schichten auszugießen und dann zu trocknen. Auf diese Weise können Folien erhalten werden.

Die warme Lösung kann auch zu Strängen gegossen und dann getrocknet werden.

Es ist auch möglich und gegebenenfalls vorteilhaft, das getrocknete Produkt in gewünschter Weise weiterzuverarbeiten; beispielsweise können durch Zerkleinern der Folien oder Stränge Granulate oder Pulver hergestellt werden.

Die auf diese Weise hergestellten Folien, können als Wundauflage, Hautersatz, als Defektauffüller für Weichgewebe, als Matrix für Wirkstoffe oder als Trägersubstanz für Zellen dienen.

Die nachfolgenden Beispiele sollen die erfindungsgemäßen Hydrogele erläutern, ohne daß eine Einschränkung auf diese Beispiele beabsichtigt ist.

Beispiel 1

Es werden getrennte Lösungen der Polymere in destilliertem Wasser angesetzt:

4

Lösung (1) 13 % ige MSA - Vinylmethylether-Copolymerisat-Lösung
Lösung (2) 3 % ige Carrageenan-Lösung

Die als Pulver vorliegenden Polymere werden unter Rühren bei Temperaturen von 45 - 60° C gelöst. Die getrennten Lösungen werden im Verhältnis 1 : 1 zusammen- gegeben und verrührt. Anschließend werden 4 Gew.-% Triethylenglycol, bezogen auf das Gesamtgewicht der vermischten Lösungen, zugegeben. Nach homogener Vermischung werden 0,25 Gew.-% Glycidyloxypropyltrimethoxysilan, bezogen auf das Gesamtgewicht der vermischten Lösungen, zugegeben.

Die so erhaltene Masse wird in eine flache Form gegeben, so daß die Füllhöhe 5 mm beträgt. Nach Trocknung bei Raumtemperatur wird eine ca. 0,5 mm starke transparente Folie mit folgenden Kennwerten erhalten:

| | | |
|---|---|---|
| Wasserdampfdurchlässigkeit | 2.100 | $\dfrac{g}{m^2 * d}$ |
| Quellwert | 5.000 | % |
| Höchstzugkraft | 34 | N |
| Reißdehnung | 58 | % |
| Biegesteifigkeit | 0,016 | Nm |

Die so erhaltene Folie ist strahlensterilisierbar, ohne daß ihre physikalischen oder physiologischen Eigenschaften beeinträchtigt würden.

Beispiel 2

Aus pulverisiertem Carrageenan wird unter Rühren und bei Erwärmen auf 45° - 60° C eine 3 % ige Lösung in destilliertem Wasser hergestellt. Nachdem sich das Carrageenan vollständig gelöst hat, werden 4 Gew.-% Glycerin, bezogen auf das Gesamtgewicht des Reaktionsansatzes, zugegeben. Nach Homogenisieren werden zuletzt 0,5 Gew.-% 3-Aminopropyltriethoxysilan zugefügt und verrührt. Die so erhaltene Masse wird in eine flache Form gegeben, so daß die Füllhöhe 5 mm beträgt. Nach Trocknung bei Raumtemperatur wird eine ca. 0,3 mm starke bernsteinfarbige transparente Folie erhalten, die einen Quellwert von 2.000 % hat.

Beispiel 3

7,5 Gew.-% Polyvinylalkohol eines mittleren Molekulargewichts von 36.000 Dalton, 3,0 Gew.-% Glycerin (84 - %ig) und 89,5 Gew.-% destilliertes Wasser werden bei 85° C verrührt.

Die Lösung wird in zwei gleiche Mengen, Ansatz (a) und (b), unterteilt. Zu Ansatz (b) werden 0,5 Gew.-% 3-Aminopropyl-triethoxysilan, bezogen auf die Gesamtmenge von Ansatz (b) zugefügt. Ansatz (a) erhält diesen Zusatz nicht.

Beide Ansätze werden getrennt weiterbehandelt. Die ca. 45° C warmen Ansätze werden durch ein Mulltuch gesiebt und auf Glasplatten gegossen. Die Ansätze werden ca. 2 Stunden lang bei Raumtemperatur entgast und danach ca. 20 Stunden lang bei 80° C im Umlufttrockenschrank getrocknet.

Von den so erhaltenen Folien werden Stücke der Abmessungen

Länge 15 cm
Breite 2 cm
Stärke 0,15 mm ausgeschnitten.

Die Reißfestigkeit und die Reißdehnungsmessung wurden mit einer Frank Universalprüfmaschine 679 ermittelt.

### Reißfestigkeit

Ansatz (a) $\quad$ 7,1 $\quad -----\quad \dfrac{N}{mm^2}$ .

Ansatz (b) $\quad$ 21,1 $\quad -----\quad \dfrac{N}{mm^2}$

### Wasserdampfdurchlässigkeit

Ansatz (a) $\quad$ 3.659 $\quad -----\quad \dfrac{g}{m^2*d}$

Ansatz (b) $\quad$ 2.952 $\quad -----\quad \dfrac{g}{m^2*d}$

### Wassergehalt

Ansatz (a) $\quad$ 28,50 $\quad$ %

Ansatz (b) $\quad$ 18,70 $\quad$ %

Beispiel 4

7,5 Gew.-% Polyvinylalkohol eines mittleren Molekulargewichtes von 40.000 Dalton, 3,0 Gew.-% Glycerin (84 %ig) und 89,5 Gew.-% destilliertes Wasser werden bei 85° C verrührt.
Die Lösung wird in zwei gleiche Mengen, Ansatz (c) und (d), unterteilt. Zu Ansatz (d) werden 0,5 Gew.-% 3-Aminopropyl-triethoxysilan, bezogen auf die Gesamtmenge von Ansatz (d) zugefügt. Ansatz (c) erhält diesen Zusatz nicht.

Beide Ansätze werden getrennt weiterbehandelt. Die ca. 45° C warmen Ansätze werden durch ein Mulltuch gesiebt und auf Glasplatten gegossen. Die Ansätze werden ca. 2 Stunden lang bei Raumtemperatur entgast und danach ca. 20 Stunden lang bei 80° C im Umlufttrockenschrank getrocknet.

Von den so erhaltenen Folien werden Stücke der Abmessungen

Länge 15 cm
Breite 2 cm
Stärke 0,15 mm
ausgeschnitten.

Die Reißfestigkeit und die Reißdehnungsmessung wurden mit einer Frank Universalprüfmaschine 679 ermittelt.

### Reißfestigkeit

$$\text{Ansatz (c)} \quad 17,4 \quad \frac{N}{mm^2}$$

$$\text{Ansatz (d)} \quad 27,5 \quad \frac{N}{mm^2}$$

### Wasserdampfdurchlässigkeit

$$\text{Ansatz (c)} \quad 3.904 \quad \frac{g}{m^2 * d}$$

$$\text{Ansatz (d)} \quad 3.455 \quad \frac{g}{m^2 * d}$$

### Wassergehalt

$$\text{Ansatz (c)} \quad 29,10 \quad \%$$

$$\text{Ansatz (d)} \quad 19,90 \quad \%$$

Beispiel 5

7,5 Gew.-% Polyvinylalkohol eines mittleren Molekulargewichtes von 80.000 Dalton, 3,0 Gew.-% Glycerin (84 %ig) und 89,5 Gew.-% destilliertes Wasser werden bei 850°C verrührt.

Die Lösung wird in zwei gleiche Mengen, Ansatz (e) und (f), unterteilt. Zu Ansatz (f) werden 0,5 Gew.-% 3-Aminopropyl-triethoxysilan, bezogen auf die Gesamtmenge von Ansatz (f) zugefügt. Ansatz (e) erhält diesen Zusatz nicht.

Beide Ansätze werden getrennt weiterbehandelt. Die ca. 45°C warmen Ansätze werden durch ein Mulltuch gesiebt und auf Glasplatten gegossen. Die Ansätze werden ca. 2 Stunden lang bei Raumtemperatur entgast und danach ca. 20 Stunden lang bei 80°C im Umlufttrockenschrank getrocknet.

Von den so erhaltenen Folien werden Stücke der Abmessungen

Länge 15 cm
Breite 2 cm
Stärke 0,15 mm ausgeschnitten.

Die Reißfestigkeit und die Reißdehnungsmessung wurden mit einer Frank Universalprüfmaschine 679 ermittelt.

Reißfestigkeit

Ansatz (e)    34,8    $\dfrac{N}{mm^2}$

Ansatz (f)    37,5    $\dfrac{N}{mm^2}$

Wasserdampfdurchlässigkeit

Ansatz (e)    3.979    $\dfrac{g}{m^2 \ast d}$

Ansatz (f)    3.488    $\dfrac{g}{m^2 \ast d}$

Wassergehalt

Ansatz (e)    30,70    %

Ansatz (f)    19,60    %

**Ansprüche**

1. Vernetzte Hydrogele, insbesondere für medizinische Anwendungen, dadurch erhältlich, daß natürliche oder synthetische makromolekulare Verbindungen mit hydrophilen Gruppen durch Alkoxysilylverbindungen bei pH-Werten im neutralen bis sauren Bereich in wäßrigem Medium vernetzt werden, wobei unter hydrophilen Gruppen
Hydroxylgruppen ( -OH),
Carboxylgruppen ( -COOH),

Amidgruppen ( -CONRR´, mit R,R´ gewählt aus H, Methyl, Ethyl),
Aminogruppen (primäre, sekundäre, tertiäre sowie quaternäre) und
Sulfonsäuregruppen
zu verstehen sind.

2. Hydrogele nach Anspruch 1, dadurch gekennzeichnet, daß die Alkoxysilylverbindungen gewählt werden aus der Gruppe der Verbindungen der folgenden allgemeinen Formel:
$HRN-(CH_2-CH_2-NR´)_n-(CH_2)_m-Si(OR´´)_3$
mit R, R´ gewählt aus der Gruppe H, Methyl, Ethyl und R´´ gewählt aus der Gruppe Methyl, Ethyl
wobei n Werte von 0 - 5
und m Werte von 1 - 4 annehmen kann.

3. Hydrogele nach Anspruch 1, dadurch gekennzeichnet, daß die Alkoxysilylverbindungen gewählt werden aus der Gruppe aus 3-Aminotriethoxysilan, N-Aminoethyl-aminopropyltrimethoxysilan, "Triaminosilan" ($H_2N$-$CH_2-CH_2-NH-CH_2-CH_2-NH-CH_2-CH_2-CH_2-Si$ $(OCH_3)_3$), N-Methyl-3-aminopropyltrimethoxysilan, 3-Ureidopropyltriethoxysilan,
3-(4,5-Dihydroimidazol-1-yl)-propyltriethoxysilan,
3-Methacryloxypropyltriethoxysilan,
3-Glycidyloxypropyltrimethoxysilan,
Vinyltriethoxysilan,
Vinyltrimethoxysilan,
Tetramethoxysilan,
Tetraethoxysilan .

4. Hydrogele nach einem der Ansprüche 1 - 3, dadurch gekennzeichnet, daß die makromolekularen Verbindungen mit hydrophilen Gruppen gewählt werden aus der Gruppe aus
Gelatine, löslichem Kollagen, Polyvinylalkohol, Poly-2-hydroxyethylmethacrylat, partiell verseiftem Polyvinylacetat, Methylcellulose, Hydroxypropylmethylcellulose, Hydroxyethylcellulose, Dextran, Carrageenan, Alginate, Maleinsäureanhydrid-Vinylether-Copolymerisate, Chitosan, Carboxymethyl-Chitin, Polyacrylsäure und/oder Polymethacrylsäure Homo- oder Copolymerisate jeweils allein oder in Mischung.

5. Hydrogele nach einem der Ansprüche 1 - 4, dadurch gekennzeichnet, daß die makromolekularen Verbindungen mit hydrophilen Gruppen gewählt werden aus der Gruppe der Polyvinylalkohole mit mittleren Molekulargewichten von 15.000 - 150.000 Dalton.

6. Hydrogele nach einem der Ansprüche 1 - 5, dadurch gekennzeichnet, daß sie Weichmacher enthalten, gewählt aus der Gruppe aus flüssigen Polyolen , insbesondere Glycerin, Ethylenglycol, flüssige Polyethylenglycole, insbesondere Propylenglycol-1,2, flüssige Polypropylenglycole-1,2 verschiedener Molmasse, Propylenglycol-1,3, flüssige Polypropylenglycole-1,3 verschiedener Molmasse,
Zitronensäuretriethyl- oder trimethylester,
Milchsäuremethyl- oder -ethylester,
Glycolsäuremethyl- oder -ethylester oder
2-Ethyl-2-(hydroxymethyl)-1,3-propandiol.

7. Hydrogele nach einem der Ansprüche 1 - 6, dadurch gekennzeichnet, daß als Katalysator für die Vernetzungsreaktion Säuren, insbesondere gewählt aus der Gruppe aus
Phosphorsäure, Salzsäure, Schwefelsäure, Essigsäure, Milchsäure, Zitronensäure, Ascorbinsäure, Salicylsäure oder der organischen Polymere mit einem Gehalt an Carboxylgruppen verwendet werden.

8. Hydrogele nach einem der Ansprüche 1 - 7, erhältlich aus
40 - 95 Gew.-% an makromolekularen Verbindungen mit hydrophilen Gruppen
4 - 60 Gew.-% an Weichmachern
0,1 - 5,0 Gew.-% an Alkoxysilylgruppenhaltigen Vernetzern
0 - 5,0 Gew.-% an Säuren,
jeweils bezogen auf die wasserfreie Mischung.

9. Hydrogele nach einem der Ansprüche 1 - 8 dadurch gekennzeichnet, daß sie als Folien, Blätter, Stränge, Pulver, Granulate oder Schäume vorliegen.

10. Verfahren zur Herstellung vernetzter Hydrogele nach einem der Ansprüche 1 - 9, dadurch gekennzeichnet, daß zunächst die makromolekulare Substanz mit hydrophilen Gruppen in Wasser unter Rühren bei 40 - 90° C gelöst wird, die anderen Bestandteile außer dem Vernetzer in beliebiger Reihenfolge zugegeben werden, der Vernetzer als letztes zugegeben wird und nach Abklingen der Vernetzungsreaktion das rohe Hydrogel bei Temperaturen von Raumtemperatur - 95° C, vorzugsweise bei Temperaturen von 60 - 95° C getrocknet wird.

11. Verwendung eines Hydrogels nach einem der Ansprüche 1 - 9 als Wundauflage oder -bedeckung, als Defektauffüller für Weichgewebe, als Wirkstoffmatrix oder als Basis für die Besiedlung mit autologen oder

heterologen Hautzellen.